# EUROPEAN PATENT APPLICATION

(11) **EP 4 825 413 A1**
(43) Date of publication of application: **21.10.2026**
(21) Application number: 25305532.1
(22) Date of filing: 14.04.2025
(51) Int. Cl.: A61M 5/178

(54) **TOOL FOR ALIGNMENT AND ENGAGEMENT OF A PLUNGER ROD WITH A STOPPER OF A PRE-FILLED SYRINGE**

(71) Applicant: Becton Dickinson France, 38800 Le Pont-de-Claix (FR)
(72) Inventor: VIAT, Laurent, 38430 Moirans (FR); GAGLIANO, Julien, 38240 MEYLAN (FR)
(74) Representative: Germain Maureau

(57) **Abstract**

Provided herein is a tool for attaching a plunger rod to a stopper. The tool includes a plunger rod shell that retains the plunger rod, a lower tool portion that retains the syringe barrel, and an upper tool portion. The upper tool portion includes guide rails, a bottom support member movable vertically on the guide rails and that retains the plunger rod shell therein, a top support member movable vertically on the guide rails and including a weight support pin movable between a raised position and a lowered position to selectively contact the plunger rod, and weights positionable on the weight support pin. The upper tool portion is movable to bring the plunger rod into contact with the stopper such that, with the weight support pin in the lowered position, the weights apply a downward force to the plunger rod to assist in engaging the plunger rod with the stopper.

## Description

### BACKGROUND OF THE INVENTION

### Field of the Invention

The present disclosure relates generally to pre-filled syringes and, more particularly, to a tool employed for the assembly of pre-filled syringes, with the tool aiding in the alignment and engagement of a plunger rod of the syringe with a stopper of a stoppered syringe barrel.

### Description of Related Art

Medical injection devices, such as syringes, are used in a variety of environments for administering fluids, such as medications or other drugs, to a patient. Syringes may be provided as pre-filled syringes, which provide the convenience of rapidly delivering the liquid therein to a patient without the need to first aspirate the medication from another container and meter its volume. A syringe will typically include a glass (or plastic) syringe barrel with an opening at a distal end and a plunger assembly inserted through the opposite proximal end of the barrel. The distal end of the syringe barrel may include a tip through which the opening is formed. In some embodiments, the tip may be configured as a luer connection that enables a transfer of fluid out from or into the syringe barrel, while in other embodiments the tip may be configured as a hub that retains a needle therein for the syringe. The plunger assembly typically includes an elongated plunger rod extending out of the barrel and a plunger head or stopper disposed at the distal end of the plunger rod. The stopper is typically made of elastomeric material and is adapted to ensure the container closure integrity of a syringe when the stopper is inserted into the syringe. The plunger assembly is movable within the syringe barrel to force fluid out from an inner volume of the syringe barrel and/or to aspirate fluid into the inner volume of the syringe barrel - with the stopper sliding through the barrel when the plunger assembly is actuated by a user.

As known in the art, manufacturing and assembly of a pre-filled syringe is performed by first filling an empty syringe barrel with a desired pharmaceutical composition, then stoppering the filled syringe barrel under vacuum or by vent tube or a combination of both. After stoppering of the syringe barrel and positioning of the stopper at a desired location within the syringe barrel, a plunger rod is then attached and secured to the stopper, to enable subsequent actuation and use of the syringe to dispense the pharmaceutical composition out from the syringe barrel. According to embodiments, the plunger rod may be attached to the stopper via a screwing engagement of a plunger rod head with a cavity of the stopper or via a pushing of a plunger rod head into a cavity of the stopper.

With regard to the attaching and securing of the plunger rod to the stopper during assembly of a pre-filled syringe, it is recognized that proper alignment of the plunger rod with the stopper, as well as application of a proper securing force to the plunger rod, is desirable to ensure proper attachment of the plunger rod with the stopper. As one example, if the plunger rod is not properly aligned with the stopper when attaching the plunger rod to the stopper, it is possible for forces to be applied to the stopper that can cause the stopper to tilt within the syringe barrel, which can lead to issues with container closure integrity of the syringe. As another example, if an insufficient securing force is applied to the plunger rod when attaching the plunger rod to the stopper, it is possible that the plunger rod may not be properly secured to the stopper, which can lead to detaching of the plunger rod from the stopper during use of the syringe, such as when the plunger rod is retracted proximally relative to the syringe barrel.

Accordingly, a need exists in the art for a tool that aids in the alignment and engagement of a plunger rod and a stopper of a stoppered syringe barrel during assembly of a pre-filled syringe. The tool should ensure proper alignment of the plunger rod with the stopper. The tool should also allow for application of a variable force when attaching the plunger rod to the stopper. The tooling should still further enable assembly of various types/sizes of syringes.

### SUMMARY OF THE INVENTION

Provided herein is a tool for attaching a plunger rod to a stopper positioned within a syringe barrel of a pre-filled syringe. The tool comprises a plunger rod shell including a pair of half-shells securable together to retain the plunger rod therebetween, a lower tool portion configured to receive and retain the syringe barrel therein, and an upper tool portion. The upper tool portion includes a frame comprising a pair of spaced apart guide rails each including a track formed therein and a bottom support member mounted on the guide rails and movable vertically within the tracks thereof, the bottom support member configured to receive and retain the plunger rod shell therein, with the plunger rod shell retaining the plunger rod therein. The upper tool portion further includes a top support member mounted on the guide rails and movable vertically within the tracks thereof, the top support member including a weight support pin thereon that is movable between a raised position and a lowered position, with the weight support pin in contact with the plunger rod when in the lowered position, and one or more weights positionable on the weight support pin. The upper tool portion is movable relative to the lower tool portion, to bring the plunger rod into contact with the stopper. With the plunger rod in contact with the stopper and the weight support pin in the lowered position, the one or more weights apply a downward force to the plunger rod to assist in engaging of the plunger rod with the stopper.

In certain configurations, the top support member comprises a top frame mount positioned between the guide rails and engaged therewith and a movable bracket rotatably coupled to the top frame mount so as to be movable between an open position and a closed position, wherein the movable bracket includes an opening formed therethrough within which the weight support pin is received, with the weight support pin movable within the opening to move between the raised position and the lowered position.

In certain configurations, the top support member comprises a pair of knurled screws of knurled screws coupling the top frame mount to the guide rails, the knurled screws configured to be selectively tightened and loosened to secure the top frame mount to the guide rails at a fixed vertical location and to enable vertical movement of the top support member along the guide rails, respectively.

In certain configurations, the bottom support member comprises a bottom frame mount positioned between the guide rails and engaged therewith and a movable arm rotatably coupled to the bottom frame mount so as to be movable between an open position and a closed position, wherein the bottom frame mount and movable arm are configured to receive and retain the plunger rod shell therein when the movable arm is in the closed position.

In certain configurations, the bottom frame mount comprises a C-shaped member defining a semi-cylindrical cavity configured to receive the plunger rod shell therein, with a bottom edge of the C-shaped member including a lip formed thereon that extends into the semi-cylindrical cavity and provides a support on which the plunger rod shell rests when positioned within the semi-cylindrical cavity.

In certain configurations, the C-shaped member includes a protrusion formed thereon that extends vertically along a height of the C-shaped member, the protrusion extending inwardly into the semi-cylindrical cavity, and the movable arm includes another protrusion formed thereon that extends vertically along a height of the movable arm, the another protrusion extending inwardly into the semi-cylindrical cavity when the movable arm is in the closed position.

In certain configurations, the lip defines an opening configured to receive a top flange of the syringe barrel when the upper tool portion is moved toward the lower tool portion and the plunger rod is brought into contact with the stopper, to align the plunger rod with the stopper in multiple dimensions.

In certain configurations, each half-shell comprises a semi-circular half-shell including a slot formed therein configured to accommodate a vane of the plunger rod and a recess formed on a linear edge of the semi-circular half shell, the recess centered about the slot formed therein, with the slot thus bisecting the recess into a first recess portion and a second recess portion, wherein with the semi-circular half-shells assembled together, the slots in the semi-circular half-shells extend out in opposing directions from a center point of the plunger rod shell, and wherein with the semi-circular half-shells assembled together, the first recess portions from the semi-circular half-shells form a collective slot and the second recess portions from the semi-circular half-shells form another collective slot 76, with the collective slots formed by the first recess portions and the second recess portions extending out in opposing directions from the center point.

In certain configurations, each semi-circular half-shell comprises a channel formed therein extending through a thickness of the half-shell, and wherein the channel of each semi-circular half-shell is configured to receive therein a respective one of the protrusion or the another protrusion when the plunger rod shell is retained in the bottom support member.

In certain configurations, the pair of half-shells comprise magnets of opposing polarity that enable the half-shells to be selectively attached to each other and separated from each other.

In certain configurations, each of the one or more weights comprises a disc-shaped weight including a central hole configured to receive the weight support pin, a first channel extending between the central hole and a circumferential edge of the disc-shaped weight to allow the disc-shaped weight to be slid onto the weight support pin, and a second channel extending radially inward from the circumferential edge of the disc-shaped weight and extending partially towards the central hole, with the second channel formed on an opposite side of the disc-shaped weight from the first channel, wherein the first channel and the second channel are configured to receive therein a respective one of the protrusion or the another protrusion of the bottom support member.

In certain configurations, the one or more weights comprises a plurality of weights stackable on the weight support pin, with each of the plurality of weights having an equal mass.

In certain configurations, the downward force applied to the plunger rod is variable based on a number of the plurality of weights stacked on the weight support pin, with the downward force variable between 0.5 N and 5.0 N.

In certain configurations, the lower tool portion comprises a main housing portion that defines a receptacle within which a portion of the syringe barrel is received, an elastic gripper positioned within a portion of the receptacle adjacent an upper opening of the main housing portion, the elastic gripper configured to securely hold the syringe barrel, with an upper flange of the syringe barrel abutting a shoulder of the elastic gripper, and a clamping nut positioned at an upper end of the main housing portion and over a portion of the elastic gripper, with the clamping nut secured to the main housing portion to retain the elastic gripper in place within the main housing portion, and with the shoulder extending up through an opening of the clamping nut.

In certain configurations, the tool is configured to engage the plunger rod with the stopper via a twisting engagement of the plunger rod into the stopper.

### BRIEF DESCRIPTION OF THE DRAWINGS

FIG. 1 is a perspective view of a prefilled syringe usable with embodiments of the disclosure;
FIG. 2 is an exploded view of the prefilled syringe of FIG. 1;
FIG. 3 is a front view of a tool for assembly of a prefilled syringe, such as the prefilled syringe of FIG. 1, according to a non-limiting embodiment described herein;
FIG. 4 is a perspective view of a plunger rod shell included in the tool of FIG. 3, with the plunger rod shell in a disassembled state, according to a non-limiting embodiment described herein;
FIG. 5 shows the plunger rod shell of FIG. 4 in an assembled state and with a plunger rod secured therein;
FIG. 6 is a perspective view of an upper tool portion included in the tool of FIG. 3, according to a non-limiting embodiment described herein;
FIG. 7 is a perspective view of the upper tool portion of FIG. 6, with the upper tool portion in a first configuration;
FIG. 8 is a perspective view of the upper tool portion of FIG. 6, with the upper tool portion in a second configuration;
FIG. 9 is a perspective view of the upper tool portion of FIG. 6, with the upper tool portion in a third configuration;
FIG. 10 is a front view of a lower tool portion included in the tool of FIG. 3, according to a non-limiting embodiment described herein;
FIG. 11 is a cross-sectional view of the lower tool portion of FIG. 10;
FIG. 12 is a front view of the tool of FIG. 3, showing the tool in a first operational state;
FIG. 13 is a front view of the tool of FIG. 3, showing the tool in a second operational state; and
FIG. 14 is a front view of the tool of FIG. 3, showing the tool in a third operational state.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

In the present disclosure, the distal end of a component or of a device means the end furthest away from the hand of the user and the proximal end means the end closest to the hand of the user, when the component or device is in the use position, i.e., when the user is holding a syringe in preparation for or during use. Similarly, in this application, the terms "in the distal direction" and "distally" mean in the direction toward the distal tip of the syringe, and the terms "in the proximal direction" and "proximally" mean in the direction opposite the direction of the distal tip of the syringe.

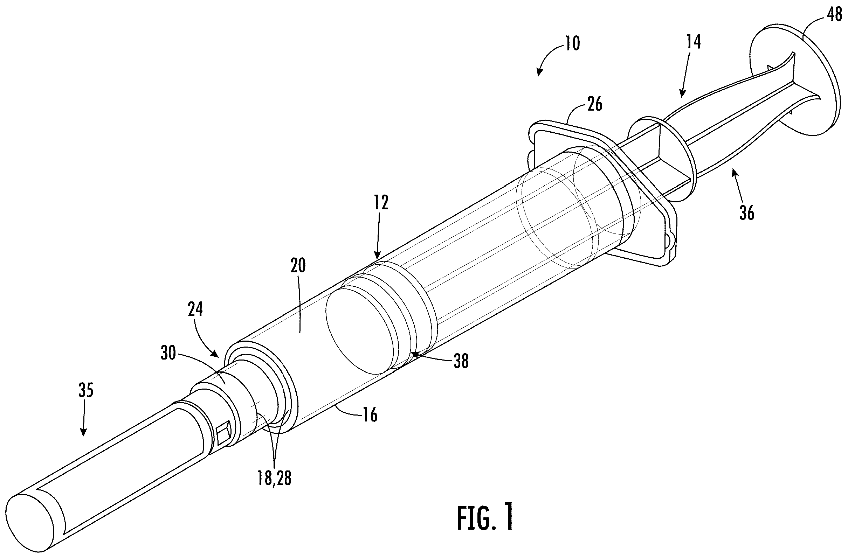

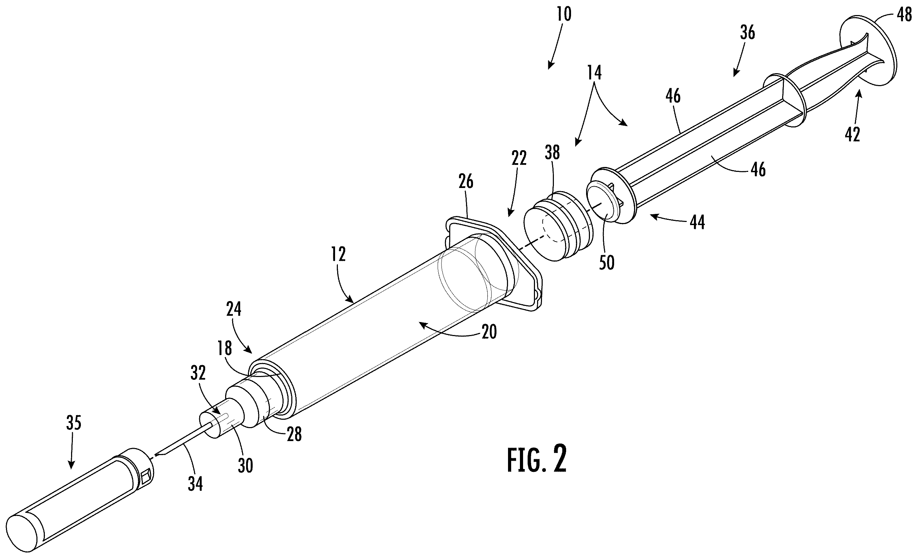

Referring to FIGS. 1 and 2, shown is a non-limiting embodiment of a medical injection device 10, such as a prefilled syringe for example, with which aspects or embodiments of the disclosure may be implemented. While the medical injection device is shown and described hereafter as a syringe 10, it is recognized that other medical injection devices (e.g., cartridges) may incorporate aspects of the disclosure, as described in detail here below.

As shown in FIGS. 1 and 2, the syringe 10 generally includes a syringe barrel 12 and a plunger assembly 14. The plunger assembly 14 is movable within the syringe barrel 12 along a longitudinal axis to an advanced position to facilitate administering of an injectable fluid (e.g., medication) to a patient, for example. The syringe barrel 12 is formed of a generally cylindrical outer wall 16 and an end member 18 that collectively define a chamber 20 for retaining fluid therein. The syringe barrel 12 includes an open proximal end 22 configured to receive the plunger assembly 14 therein and a distal end 24 at which end member 18 is positioned. The proximal end 22 of the syringe barrel 12 may include a flange 26 to facilitate handling and positioning of the syringe 10 and to maintain the relative position of the syringe barrel 12 with respect to the plunger assembly 14 during medication administration. At the distal end 24, the end member 18 may include a shoulder 28 which narrows with respect to the cylindrical outer wall 16, as well as a hub portion 30 extending out distally from shoulder 28. The hub portion 30 is formed as a partially hollow member that defines a channel 32 therethrough in fluid communication with the chamber 20. In some embodiments, a needle 34 is attached to the hub portion 30 within channel 32, such as by being glued or otherwise secured to the hub portion 30. In other embodiments, it is recognized that - rather than needle 34 - syringe 10 could instead include a luer connection formed at the distal end 24 of syringe barrel 12 that enables the syringe 10 to be directly coupled to a luer connection of an associated medical device or component, to provide for the direct transfer of fluids therebetween.

In some embodiments, syringe 10 may further include a tip cap 35 that may be coupled to the hub portion 30 of syringe barrel 12. While shown in FIGS. 1 and 2 as a needle shield that is used to protect the needle 34, tip cap 35 could instead be configured as a cap that covers a luer connection.

The plunger assembly 14 of syringe 10 is formed of an elongate plunger rod 36 (more generally "plunger 36," as used hereafter) and a plunger head or stopper 38. The plunger 36 may include a main body 40 extending between a plunger proximal end 42 and a plunger distal end 44. In some embodiments, the main body 40 may include a plurality of elongate vanes or walls 46 extending axially along a length thereof between the plunger proximal end 42 and the plunger distal end 44. A thumb press 48 is positioned at the plunger proximal end 42 that may be engaged by a thumb (or other finger) of the user to apply a distally directed force to the plunger assembly 14 to move the plunger 36 with respect to the syringe barrel 12. An extension member 50 is positioned at the plunger distal end 44 that is configured to mate with the stopper 38. In some embodiments, the extension member 50 may comprise a threaded member configured to mate with a corresponding threaded receptacle or cavity formed in stopper 38.

The stopper 38 of plunger assembly 14 is positioned at the plunger distal end 44 so as to be movable along with the plunger 36 within the chamber 20 of syringe barrel 12. The stopper 38 may be made from a material that is different from the material of the plunger 36 and that is capable of forming a tight seal with the syringe barrel 12 as it is advanced therethrough. As indicated above, the stopper 38 includes a cavity or receptacle 52 therein that is sized and configured to receive the extension member 50 of plunger 36, with the extension member 50 coupling with the receptacle 52 via a threaded engagement or press fit connection, for example, to secure the stopper 38 to the plunger 36.

As indicated above, the syringe 10 may be provided as a prefilled syringe that contains an injectable fluid therein. In manufacturing/assembly of a prefilled syringe, the syringe barrel 12 is filled with a desired pharmaceutical composition and a "stoppering" process is then performed where the stopper 38 is inserted and positioned within the syringe barrel 12, such as under vacuum or by vent tube or a combination of both. After stoppering, the plunger rod 36 may then be secured to the stopper 38 to complete manufacturing/assembly of the prefilled syringe 10.

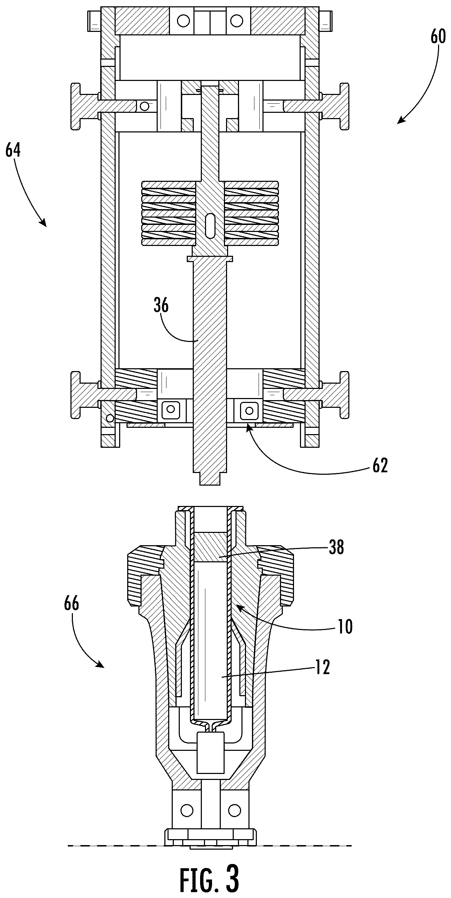

Referring now to FIGS. 3-14, according to aspects of the disclosure, a tool 60 is provided by which the plunger rod 36 may be assembled with and secured to stopper 38. The tool 60 includes a plunger rod shell 62 for securing the plunger rod 36, an upper tool portion 64 that holds the plunger rod shell 62 and plunger rod 36, and a lower tool portion 66 that holds the stoppered syringe barrel 12 (i.e., syringe barrel 12 with stopper 38 therein). As explained in further detail below, the tool 60 provides for proper alignment of the plunger rod 36 with the stopper 38 during the assembly/mating process and also provides for application of a variable force when attaching the plunger rod 36 to the stopper 38.

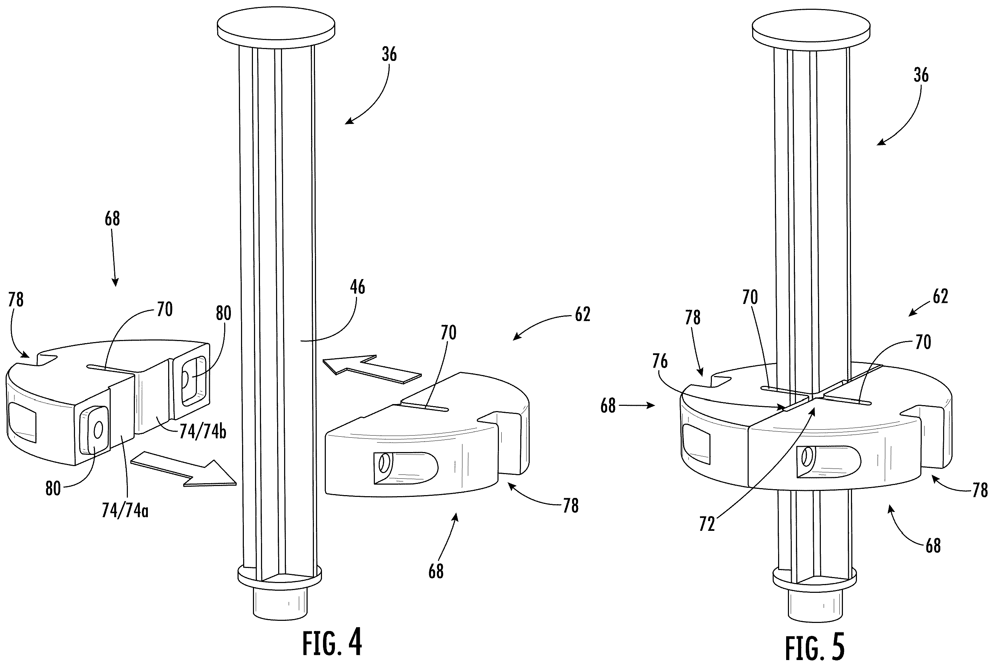

Referring to FIGS. 4 and 5, the plunger rod shell 62 is shown in greater detail. The plunger rod shell 62 may be composed of two identical half-shells 68 each having a semi-circular construction. Each half-shell 68 includes a slot 70 formed therein configured to accommodate a vane 46 of the plunger rod 36. With the half-shells 68 assembled together, the slots 70 in the half-shells 68 extend out in opposing directions from a center point 72 of the plunger rod shell 62, such that the slots 70 may accommodate a pair of opposed vanes 46 of the plunger rod 36.

Each of half-shells 68 further includes a notch or recess 74 formed on the linear edge/surface thereof. The recess 74 on each half-shell 68 may be centered about the slot 70 formed therein, with the slot 70 thus bisecting the recess 74 into a first recess portion 74a and a second recess portion 74b. With the half-shells 68 assembled together, the first recess portions 74a from the two half-shells 68 form a collective slot 76 and the second recess portions 74b from the two half-shells 68 form another collective slot 76, with the collective slots 76 formed by the first recess portions 74a and the second recess portions 74b extending out in opposing directions from the center point 72 of the plunger rod shell 62, such that the collective slots 76 may accommodate another pair of opposed vanes 46 of the plunger rod 36.

In some embodiments, each of the half-shells 68 may further include a channel 78 formed therein on a circumferential edge of the half-shell 68. The channel 78 may comprise a U-shaped channel that is formed through a thickness of the half-shell 68. The channels 78 may be formed such that, with the half-shells 68 assembled together, the channels 78 are formed on opposing sides of the plunger rod shell 62.

The half-shells 68 may be selectively attached to each other and separated from each other via suitable securing mechanisms included in the half-shells 68. In an exemplary embodiment, and as shown in FIG. 4, the half-shells 68 include magnets 80 of opposing polarity that enable the half-shells 68 to be selectively attached to each other and separated from each other.

In use of plunger rod shell 62, the pair of half-shells 68 may be attached/connected to each other, with the plunger rod 36 secured between the half-shells 68. As shown in FIG. 5, in some embodiments, the plunger rod shell 62 may be secured to plunger rod 36 at a location on the bottom half of the plunger rod 36 - with a majority of a length of the plunger rod 36 thus extending up from the plunger rod shell 62.

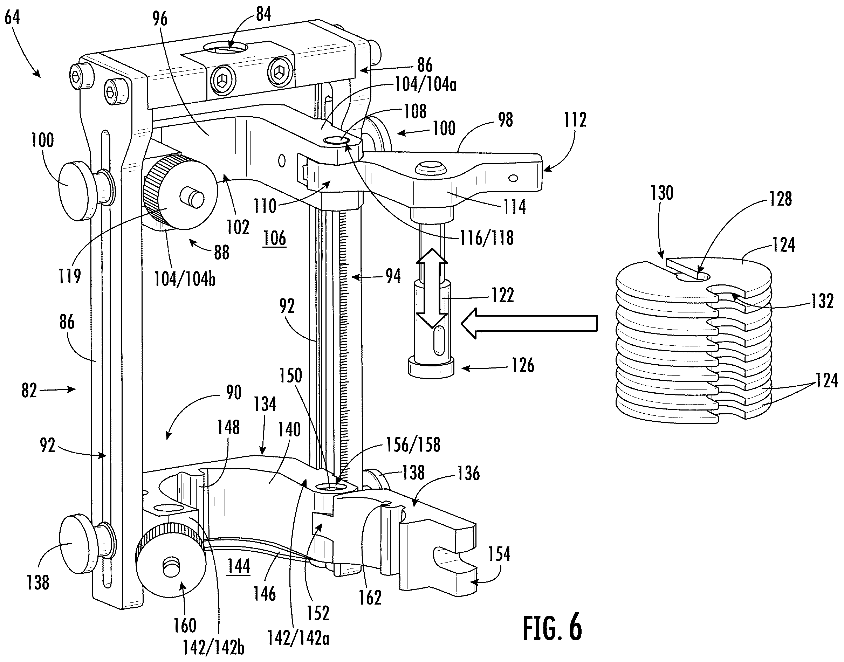

Referring next to FIGS. 6-9, the upper tool portion 64 is shown in greater detail. The upper tool portion 64 generally includes a frame 82 having a top plate 84 and guide rails 86 attached to and extending down from the top plate 84. The tool 60 also includes a top support member 88 and a bottom support member 90 mounted on the guide rails 86 and movable therealong relative to the frame 82.

As shown in FIGS. 6-9, the frame 82 of tool 60 is configured as a generally open frame 82 that provides for visibility of and access to the plunger rod 36 when positioned in/on the tool 60. The top plate 84 may comprise a rectangular plate to which the guide rails 86 are affixed at opposing ends thereof, with the guide rails 86 secured to the top plate 84 via suitable fasteners. Each of guide rails 86 includes a groove or track 92 formed therein running along a length of the guide rail 86, within which the top support member 88 and bottom support member 90 may be vertically translated. In some embodiments, one or both of the guide rails 86 may include markings 94 thereon that provide a ruler via which a vertical positioning of the top support member 88 and/or bottom support member 90 on the guide rails 86 may be determined. Via a controlled vertical positioning of the top support member 88 and bottom support member 90 on the guide rails 86, the upper tool portion 64 may be specifically configured to accommodate a plurality of sizes of plunger rods 36 therein, such that syringes of various sizes/volumes may be assembled via use of tool.

The top support member 88 may be composed of a top frame mount 96 and a movable bracket 98 that is rotatably coupled to the top frame mount 96. The top frame mount 96 is positioned between the guide rails 86 and is secured thereto via a pair of knurled screws 100 that may be selectively tightened and/or loosened to secure the top frame mount 96 to the guide rails 86 at a fixed vertical location and to enable vertical movement of the top support member 88 along the guide rails 86, respectively.

The top frame mount 96 may comprise a central member 102 and two end members 104 (i.e., a first end member 104a and a second end member 104b) at opposing sides of the central member 102. The two end members 104 extend out orthogonally from the central member 102 to roughly define a cavity 106 therein. The movable bracket 98 is rotatably coupled to the first end member 104a, such as via a pivot pin 108 for example, to provide for rotation of the movable bracket 98 between an open position and a closed position.

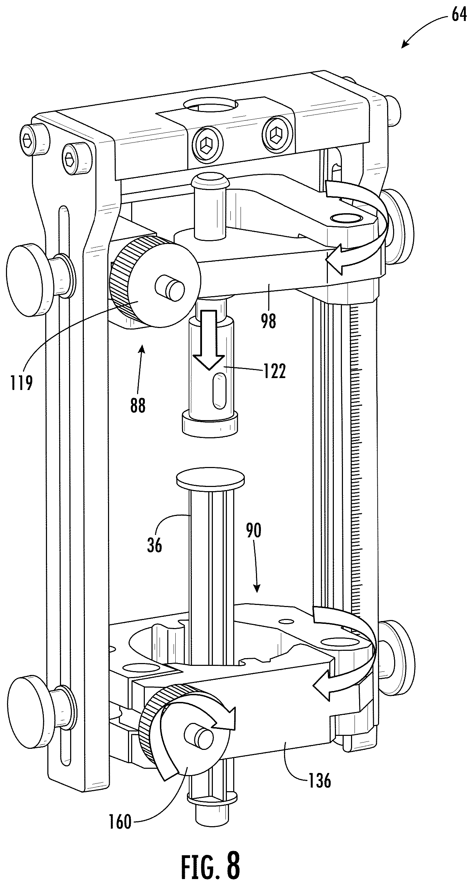

The movable bracket 98 comprises a first end 110 and a second end 112, with a central protrusion 114 that extends out horizontally from the opposing ends. The first end 110 of movable bracket 98 includes a pin hole 116 formed therein that may be aligned with a pin hole 118 in the first end member 104a of top frame mount 96, for receiving the pivot pin 108 therein to rotatably connect the movable bracket 98 to the top frame mount 96. The second end 112 of movable bracket 98 may be configured to engage with the second end member 104b of top frame mount 96 when the movable bracket 98 is rotated to its closed position. In some embodiments, second end 112 of movable bracket 98 may engage with a flanged end of the second end member 104b of top frame mount 96 when the movable bracket 98 is rotated to its closed position. In some embodiments, a retractable quick-release knurled button 119 (see FIG. 8) may be selectively added to upper tool portion 64 to secure the second end 112 of movable bracket 98 to the second end member 104b of top frame mount 96, to retain the movable bracket 98 in the closed position. The central protrusion 114 of movable bracket 98 includes an opening 120 formed therein extending between top and bottom surfaces of the movable bracket 98, with the opening 120 configured to receive a weight support pin 122 therein, as explained in further detail below.

As indicated above, a weight support pin 122 may be provided as part of upper tool portion 64, with the weight support pin 122 secured within the opening 120 of movable bracket 98 so as to be translatable vertically within the opening 120. The weight support pin 122 may be configured as an elongated pin having a top end positionable within the opening 120 and a bottom end configured to receive one or more disc-shaped weights 124 thereon. The bottom end may include an annular flange 126 formed thereon extending radially outward from a remainder of the weight support pin 122, with the flange 126 functioning to retain the weights 124 on the pin. The weight support pin 122 is movable vertically within the opening 120 between a raised position and a lowered position in order to selectively allow for the weight support pin 122 to be placed into contact with the plunger rod 36 retained by the upper tool portion 64, for assembly/engaging of the plunger rod 36 with the stoppered syringe barrel 12.

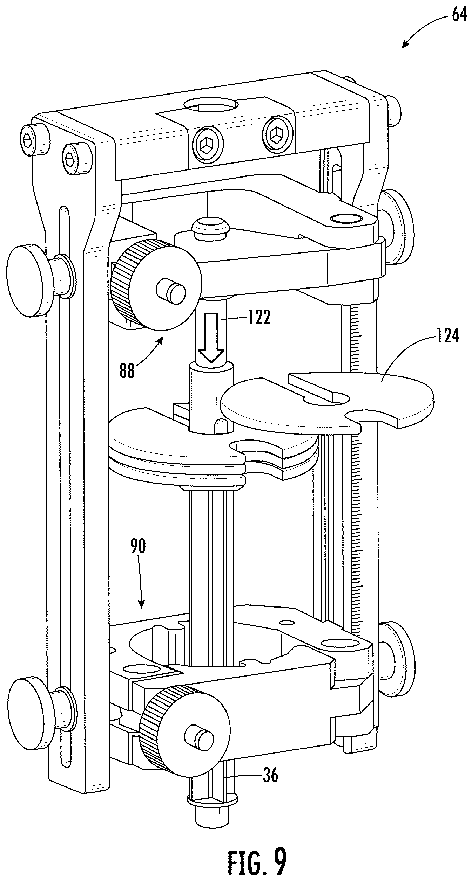

As best shown in FIGS. 6 and 9, a series of disc-shaped weights 124 may be provided for use with the upper tool portion 64, with the weights 124 being selectively addable to the weight support pin 122 to assist in engaging of the plunger rod 36 with the stoppered syringe barrel 12, as explained in further detail below. Each of the disc-shaped weights 124 comprises a central hole 128 configured to receive the weight support pin 122, along with a first channel 130 and a second channel 132 that extend vertically through an entirety of a thickness of the weight. The first channel 130 extends between the central hole 128 and a circumferential edge of the disc-shaped weight 124, in order to allow the disc-shaped weight 124 to be slid onto the weight support pin 122. The second channel 132 extends radially inward from the circumferential edge of the disc-shaped weight 124 and extends partially towards the central hole 128, with the second channel 132 formed on an opposite side of the disc-shaped weight 124 from the first channel 130. As explained further below, each of the first and second channels 130, 132 may accommodate protrusions formed on the bottom support member 90, in cases where the top support member 88 and weight support pin 122 and weights 124 are lowered relative to the bottom support member 90.

According to aspects of the disclosure, a desired number of the disc-shaped weights 124 may be selectively added onto the weight support pin 122. The disc-shaped weights 124 may be provided in a stacked arrangement on the weight support pin 122. In some embodiments, the number of disc-shaped weights 124 added to the weight support pin 122 can vary from one (1) disc-shaped weight 124 to ten (10) disc-shaped weights 124. Each of the weights 124 may have an identical mass. In some embodiments, each of the disc-shaped weights 124 may have a mass of 0.5 Newtons (N), such that a total mass of weights 124 provided on weight support pin 122 can vary from 0.5 N to 5.0 N.

The bottom support member 90 may be composed of a bottom frame mount 134 and a movable arm 136 that is rotatably coupled to the bottom frame mount 134. The bottom frame mount 134 is positioned between the guide rails 86 and is secured thereto via a pair of knurled screws 138 that may be selectively tightened and/or loosened to secure the bottom frame mount 134 to the guide rails 86 at a fixed vertical location and to enable vertical movement of the bottom support member 90 along the guide rails 86, respectively.

The bottom frame mount 134 may comprise a C-shaped member 140 having a pair of end members 142 (i.e., first end member 142a and second end member 142b) at opposing ends/sides thereof. The C-shaped member 140 roughly defines a semi-cylindrical cavity 144 therein that is configured to receive the plunger rod shell 62 therein. A bottom edge of the C-shaped member 140 includes a lip 146 formed thereon that extends into the semi-cylindrical cavity, with the lip 146 providing a support on which the plunger rod shell 62 rests when positioned within the semi-cylindrical cavity 144. In some embodiments, a protrusion 148 is formed on the C-shaped member 140 that extends vertically along a height of the C-shaped member 140. The protrusion 148 is formed along a center line of the C-shaped member 140 and extends inwardly into the semi-cylindrical cavity 144, with the protrusion 148 configured to engage the channel 130 formed in one of the half-shells 68, in order to aid in alignment and retention of the plunger rod shell 62 in bottom support member 90.

The movable arm 136 of bottom support member 90 is rotatably coupled to the first end member 142a of the bottom frame mount 134, such as via a pivot pin 150 for example, to provide for rotation of the movable arm 136 between an open position and a closed position. The movable arm 136 comprises a first end 152 and a second end 154, with an inner edge of the movable arm 136 (that faces cavity 144 when the movable arm 136 is in the closed position) that extends between the first and second ends 152, 154 having a curved profile that generally matches a curvature of the plunger rod shell 62. The first end 152 of the movable arm 136 includes a pin hole 156 formed therein that may be aligned with a pin hole 158 in the first end member 142a of the bottom frame mount 134, for receiving the pivot pin 150 therein to rotatably connect the movable arm 136 to the bottom frame mount 134. In some embodiments, the first end 152 of movable arm 136 and the first end member 142a may engage one another via a tongue and groove type connection, with the pivot pin 150 extending through the pin holes 156, 158 of the movable arm 136 and first end member 142a to enable rotation of the movable arm 136 relative to the bottom frame mount 134.

The second end 154 of movable arm 136 may be configured to engage with the second end member 142b of bottom frame mount 134 when the movable arm 136 is rotated to its closed position. In some embodiments, the second end 154 of movable arm 136 and the second end member 142b may engage one another via a tongue and groove type connection. Additionally, in some embodiments, a retractable quick-release knurled button 160 may be selectively added to bottom tool portion to secure the second end 154 of movable arm 136 to the second end member 142b of bottom frame mount 134, to retain the movable arm 136 in the closed position. The movable arm 136 may also include a protrusion 162 formed on the curved inner surface thereof - at a location between the first and second ends 152, 154. The protrusion 162 extends vertically along a height of the movable arm 136 and extends inwardly into the semi-cylindrical cavity 144 when the movable arm 136 is in the closed position, with the protrusion 162 configured to engage the channel 132 formed in one of the half-shells 68, in order to aid in alignment and retention of the plunger rod shell 62 in bottom support member 90.

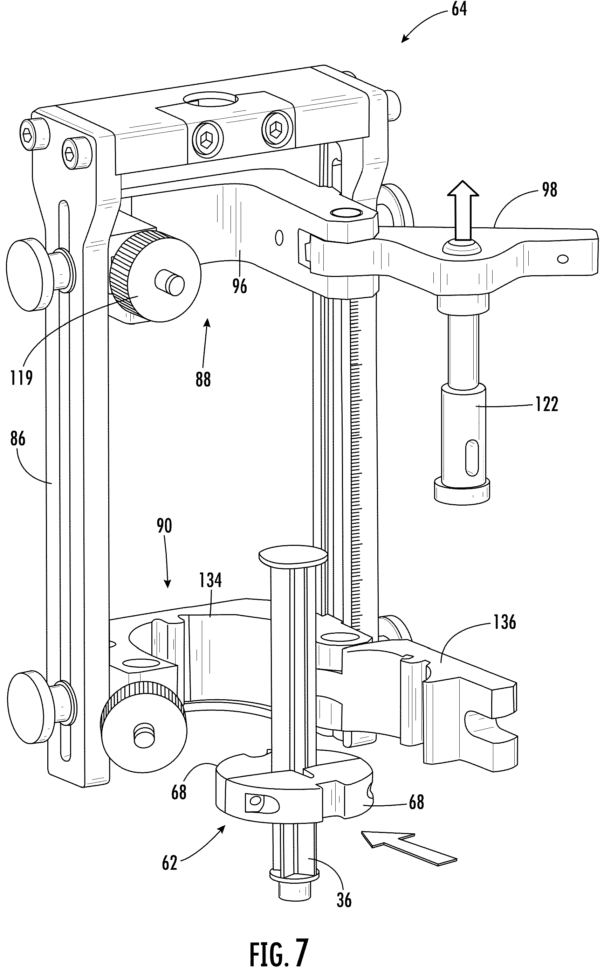

Referring to FIGS. 7-9, a loading of a plunger rod 36 and plunger rod shell 62 into upper tool portion 64 is shown and described in further detail.

As first shown in FIG. 7, the upper tool portion 64 is actuated or configured such that each of the top support member 88 and bottom support member 90 are in their open configuration, with the movable bracket 98 and movable arm 136 rotated outward and away from the top and bottom frame mounts 96, 134 to their open position. With the top support member 88 and bottom support member 90 in their open configuration, the interior region or volume of the upper tool portion 64 is open for access by a user, thereby allowing for front-loading of the plunger rod shell 62 and plunger rod 36 into the upper tool portion 64.

As further shown in FIG. 7, the plunger rod 36 is secured within the plunger rod shell 62. As previously described, half-shells 68 of the plunger rod shell 62 may be assembled together with the plunger rod 36 retained within slots 70, 76 (FIGS. 4 and 5) formed in the plunger rod shell 62. The plunger rod 36 may be mounted high within the plunger rod shell 62, such that a majority of the plunger rod 36 extends upward from the plunger rod shell 62.

In loading the plunger rod shell 62 and plunger rod 36 into the upper tool portion 64, it is recognized that the top support member 88 and bottom support member 90 may be positioned vertically on the guide rails 86 at a location appropriate for the size of the plunger rod 36 (and overall syringe 10). That is, the top support member 88 and bottom support member 90 may be raised and/or lowered to vertical locations that accommodate the plunger rod 36 in the upper tool portion 64, including providing for appropriate alignment between the weight support pin 122 and plunger rod 36 - i.e., allowing for loading of the plunger rod 36 into upper tool portion 64 with the weight support pin 122 in a raised position and for engagement between the weight support pin 122 and plunger rod 36 with the weight support pin 122 in a lowered position.

As next shown in FIG. 8, the upper tool portion 64 is actuated to move each of the movable bracket 98 and movable arm 136 from the open position to the closed position. While actuating the movable bracket 98 of the top support member 88 to its closed position, the weight support pin 122 is maintained in a raised position - to provide clearance between the weight support pin 122 and the plunger rod 36 and enable closing of the movable bracket 98. Upon the movable bracket 98 of top support member 88 and the movable arm 136 of bottom support member 90 being moved to their closed position, the movable bracket 98 and movable arm 136 may be secured in place via the addition and actuation of retractable quick-release knurled buttons 119, 160.

As next shown in FIG. 9, with the plunger rod shell 62 and plunger rod 36 loaded into upper tool portion 64 and the top support member 88 and bottom support member 90 in their closed configuration, the weight support pin 122 may be released and lowered into contact with a top end of plunger rod 36. With the weight support pin 122 lowered into contact with plunger rod 36, a desired number of disc-shaped weights 124 may be added onto the weight support pin 122 - with weights 124 being selectively added to apply a desired magnitude of downward pushing force against the plunger rod 36, for purposes of engaging the plunger rod 36 with stopper 38, as explained if further detail below. According to embodiments of the disclosure, a desired number of disc-shaped weights 124 may be added onto the weight support pin 122 to apply a pushing force of between 0.5 N and 5.0 N, as a non-limiting example.

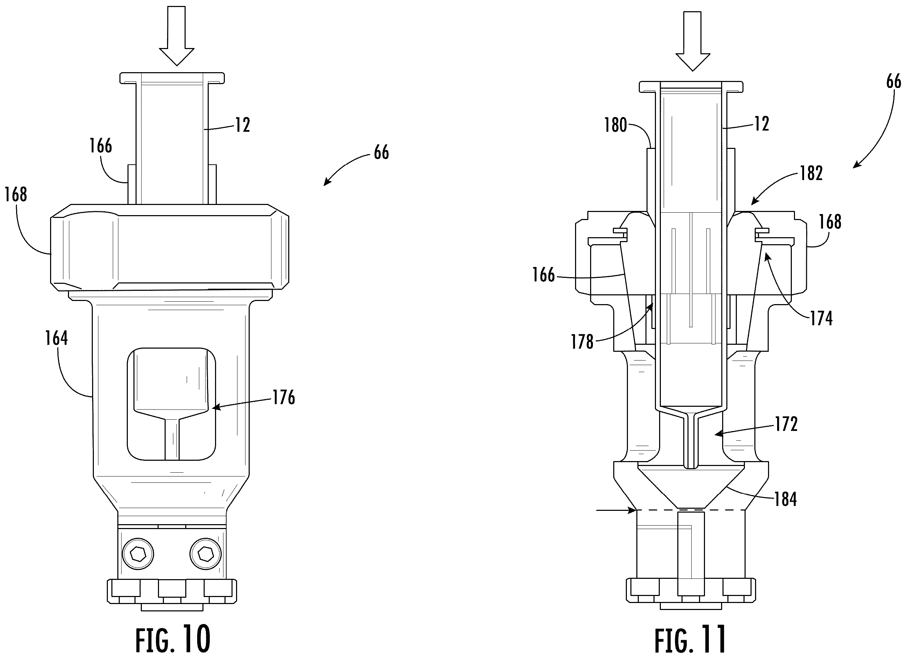

Referring now to FIGS. 10 and 11, the lower tool portion 66 is shown in greater detail. The lower tool portion 66 generally includes a main housing portion 164, an elastic gripper 166, and a clamping nut 168.

The main housing portion 164 generally defines a receptacle 172 within which a portion of the syringe barrel 12 may be received. An upper opening 174 of the main housing portion 164 provides access to the receptacle 172, for placement of the syringe barrel 12 into the receptacle 172. The main housing portion 164 may include a through-hole opening 176 formed therein, at a location between upper and lower ends of the main housing portion 164, with the through-hole opening 176 allowing for viewing of the syringe barrel 12 within the receptacle 172.

The elastic gripper 166 is positioned within a portion of the receptacle 172 adjacent the upper opening 174. The elastic gripper 166 may generally be configured to have a conical shape, with the elastic gripper 166 gradually tapering from a top end to a bottom end thereof, and with a tapering of the elastic gripper 166 generally matching a tapering of upper opening 174 of main housing portion 164, such that the elastic gripper 166 may be received and retained in the upper opening 174. A gripper opening 178 is formed through the elastic gripper 166 that is sized and configured to receive the syringe barrel 12 therein. A diameter of the gripper opening 178 may be slightly smaller than an outer diameter of the syringe barrel 12, but with the elastic gripper 166 configured to flex/deform to increase the size of the gripper opening 178 to accommodate the syringe barrel 12 therein - with the syringe barrel 12 thus being securely retained within the elastic gripper 166. An upper end of the elastic gripper 166 may provide a shoulder 180 against which the flange 26 of syringe barrel 12 may rest, to secure syringe barrel 12 in-place within the elastic gripper 166.

The clamping nut 168 is positioned at the upper end of the main housing portion 164 and over a portion of the elastic gripper 166. According to embodiments, the clamping nut 168 may be secured to the main housing portion 164 via a snap-fit engagement or a twisting, threaded engagement, as non-limiting examples. The clamping nut 168 functions to retain the elastic gripper 166 in place within the main housing portion 164, with an upper opening 182 in the clamping nut 168 allowing for an upper end of the elastic gripper 166 (i.e., shoulder 180 of elastic gripper 166) to extend up past the clamping nut 168.

In use of the lower tool portion 66, syringe barrels 12 of different size may be accommodated via a height adjustment of the lower tool portion 66. That is, with the syringe barrel 12 inserted into elastic gripper 166 and the flange 26 of syringe barrel 12 resting against the shoulder 180, a vertical positioning of the elastic gripper 166 relative to main housing portion 164 (i.e., how far elastic gripper 166 protrudes up and out from main housing portion 164) may be adjusted via actuation of clamping nut 168. That is, clamping nut 168 may be twisted by a user to vary a positioning of the clamping nut on main housing portion 164 - with the clamping nut 168 being screwed or unscrewed by the user to vertically shift (i.e., raise or lower) the clamping nut 168 relative to main housing portion 164. This height adjustment can be performed so that the end wall 18 and/or shoulder 28 of syringe barrel 12 is caused to come into contact with a support surface 184 of the main housing portion 164, to secure/support the syringe barrel 12 during engagement of the plunger rod 36 with stopper 38.

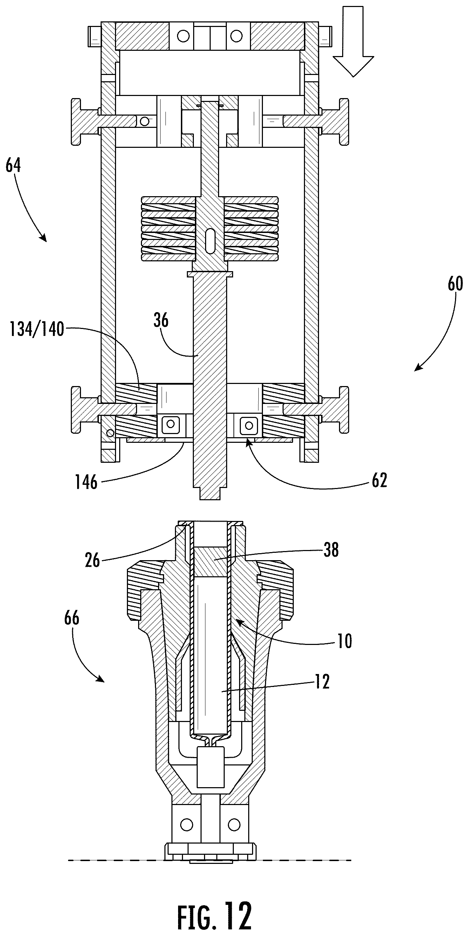

Referring now to FIGS. 12-14, operation of the tool 60 for alignment and assembly of the plunger rod 36 with the stoppered syringe barrel 12 is shown and described in further detail. Initially, it is understood that the plunger rod 36 (and plunger rod shell 62) is loaded into the upper tool portion 64 of tool 60, as shown and described previously in FIGS. 7-9, and that the syringe barrel 12 is loaded into the lower tool portion 66 of tool 60, as shown and described previously in FIGS. 10 and 11.

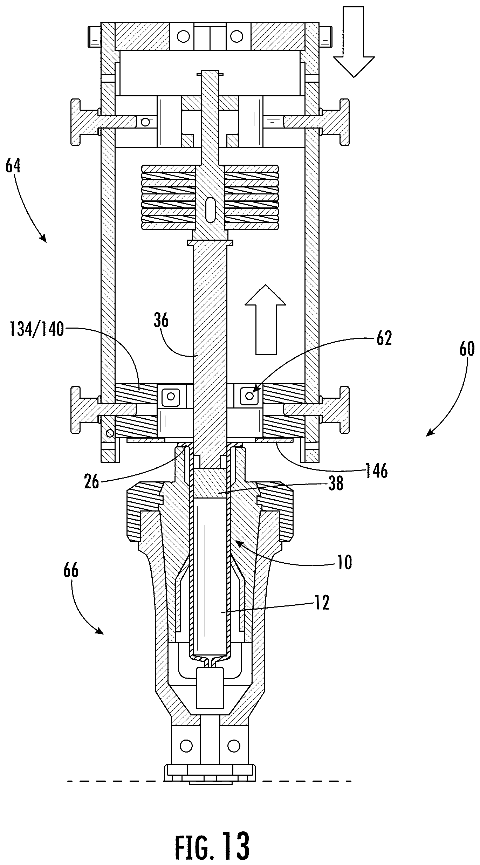

With the plunger rod 36 (and plunger rod shell 62) loaded into the upper tool portion 64 and the syringe barrel 12 loaded into the lower tool portion 66, operation of the tool 60 may proceed with lowering of the upper tool portion 64 into alignment and engagement with the lower tool portion 66, as shown in transitioning of the tool 60 from the configuration of FIG. 12 to the configuration of FIG. 13. The upper tool portion 64 may be lowered until the distal end of plunger rod 36 is brought into contact with the stopper 38 within plunger barrel 12.

Upon the plunger rod 36 being brought into contact with the stopper 38, alignment of the syringe barrel 12 and plunger rod 36 may be ensured via engaging and aligning of the syringe barrel 12 with the upper tool portion 64 and plunger rod shell 62 of tool 60, as shown in FIG. 13. In one embodiment, the top flange 26 of syringe barrel 12 may be interfit with an opening defined by the lip 146 formed on the C-shaped member 140 of bottom frame mount 134 and brought into contact with an underside of plunger rod shell 62, to ensure that the plunger rod 36 is aligned with the stopper 38 in multiple dimensions (i.e., aligned in x, y and z axes).

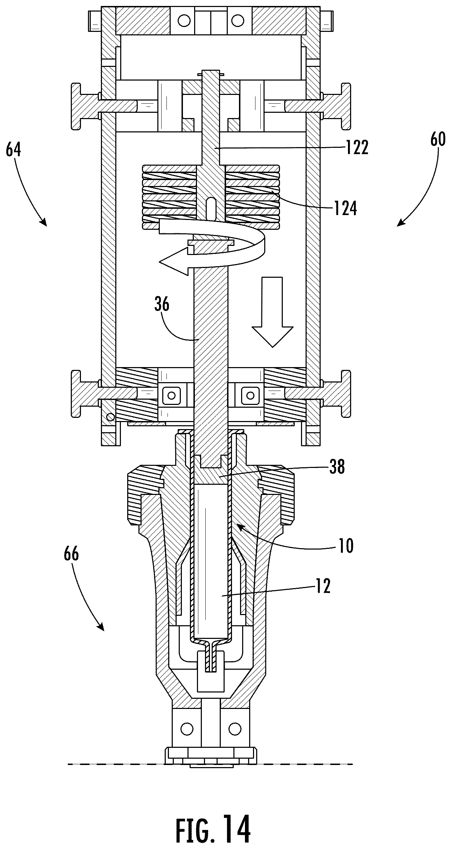

Finally, as shown in FIG. 14, the plunger rod 36 may be secured to stopper 38 via application of a twisting force/motion that screws the plunger rod 36 into the stopper 38. As the plunger rod 36 is being screwed into stopper 38, the downward force applied to plunger rod 36 by the disc-shaped weights 124 provided on weight support pin 122 aids in engaging of the plunger rod 36 with stopper 38. In some embodiments, the plunger rod 36 may be screwed into stopper 38 in an automated fashion, such as via a servo motor (not shown) applying a twisting force to plunger rod 36, while in other embodiments the plunger rod 36 may be manually screwed into stopper 38.

Beneficially, embodiments of the invention thus provide a tool for attaching a plunger rod to a stopper that is stoppered within a syringe barrel of a pre-filled syringe. The tool aids in the alignment and engagement of the plunger rod and the stopper of the stoppered syringe barrel during assembly of the pre-filled syringe. The tool ensures proper alignment of the plunger rod with the stopper in multiple dimensions and allows for the application of a variable force when attaching the plunger rod to the stopper, while accommodating assembly of various sizes of syringes

Although the present disclosure has been described in detail for the purpose of illustration based on what is currently considered to be the most practical and preferred embodiments or aspects, it is to be understood that such detail is solely for that purpose and that the present disclosure is not limited to the disclosed embodiments or aspects, but, on the contrary, is intended to cover modifications and equivalent arrangements that are within the spirit and scope of the appended claims. For example, it is to be understood that the present disclosure contemplates that, to the extent possible, one or more features of any embodiment may be combined with one or more features of any other embodiment.

## Claims

1. A tool for attaching a plunger rod to a stopper positioned within a syringe barrel of a pre-filled syringe, the tool comprising:
a plunger rod shell including a pair of half-shells securable together to retain the plunger rod therebetween;
a lower tool portion configured to receive and retain the syringe barrel therein; and
an upper tool portion comprising:
a frame comprising a pair of spaced apart guide rails each including a track formed therein;
a bottom support member mounted on the guide rails and movable vertically within the tracks thereof, the bottom support member configured to receive and retain the plunger rod shell therein, with the plunger rod shell retaining the plunger rod therein;
a top support member mounted on the guide rails and movable vertically within the tracks thereof, the top support member including a weight support pin thereon that is movable between a raised position and a lowered position, with the weight support pin in contact with the plunger rod when in the lowered position; and
one or more weights positionable on the weight support pin;
wherein the upper tool portion is movable relative to the lower tool portion, to bring the plunger rod into contact with the stopper; and
wherein with the plunger rod in contact with the stopper and the weight support pin in the lowered position, the one or more weights apply a downward force to the plunger rod to assist in engaging of the plunger rod with the stopper.

2. The tool of claim 1, wherein the top support member comprises:
a top frame mount positioned between the guide rails and engaged therewith; and
a movable bracket rotatably coupled to the top frame mount so as to be movable between an open position and a closed position;
wherein the movable bracket includes an opening formed therethrough within which the weight support pin is received, with the weight support pin movable within the opening to move between the raised position and the lowered position.

3. The tool of claim 2, wherein the top support member comprises a pair of knurled screws of knurled screws coupling the top frame mount to the guide rails, the knurled screws configured to be selectively tightened and loosened to secure the top frame mount to the guide rails at a fixed vertical location and to enable vertical movement of the top support member along the guide rails, respectively.

4. The tool of any of claims 1-3, wherein the bottom support member comprises:
a bottom frame mount positioned between the guide rails and engaged therewith; and
a movable arm rotatably coupled to the bottom frame mount so as to be movable between an open position and a closed position;
wherein the bottom frame mount and movable arm are configured to receive and retain the plunger rod shell therein when the movable arm is in the closed position.

5. The tool of claim 4, wherein the bottom frame mount comprises a C-shaped member defining a semi-cylindrical cavity configured to receive the plunger rod shell therein, with a bottom edge of the C-shaped member including a lip formed thereon that extends into the semi-cylindrical cavity and provides a support on which the plunger rod shell rests when positioned within the semi-cylindrical cavity.

6. The tool of claim 5, wherein the C-shaped member includes a protrusion formed thereon that extends vertically along a height of the C-shaped member, the protrusion extending inwardly into the semi-cylindrical cavity; and wherein the movable arm includes another protrusion formed thereon that extends vertically along a height of the movable arm, the another protrusion extending inwardly into the semi-cylindrical cavity when the movable arm is in the closed position.

7. The tool of claim 5 or claim 6, wherein the lip defines an opening configured to receive a top flange of the syringe barrel when the upper tool portion is moved toward the lower tool portion and the plunger rod is brought into contact with the stopper, to align the plunger rod with the stopper in multiple dimensions.

8. The tool of any of claims 5-7, wherein each half-shell comprises a semi-circular half-shell including:
a slot formed therein configured to accommodate a vane of the plunger rod;
a recess formed on a linear edge of the semi-circular half shell, the recess centered about the slot formed therein, with the slot thus bisecting the recess into a first recess portion and a second recess portion;
wherein with the semi-circular half-shells assembled together, the slots in the semi-circular half-shells extend out in opposing directions from a center point of the plunger rod shell; and
wherein with the semi-circular half-shells assembled together, the first recess portions from the semi-circular half-shells form a collective slot and the second recess portions from the semi-circular half-shells form another collective slot 76, with the collective slots formed by the first recess portions and the second recess portions extending out in opposing directions from the center point.

9. The tool of claim 8, wherein each semi-circular half-shell comprises a channel formed therein extending through a thickness of the half-shell, and wherein the channel of each semi-circular half-shell is configured to receive therein a respective one of the protrusion or the another protrusion when the plunger rod shell is retained in the bottom support member.

10. The tool of claim 8 or claim 9, wherein the pair of half-shells comprise magnets of opposing polarity that enable the half-shells to be selectively attached to each other and separated from each other.

11. The tool of any of claims 5-10, wherein each of the one or more weights comprises a disc-shaped weight including:
a central hole configured to receive the weight support pin;
a first channel extending between the central hole and a circumferential edge of the disc-shaped weight to allow the disc-shaped weight to be slid onto the weight support pin; and
a second channel extending radially inward from the circumferential edge of the disc-shaped weight and extending partially towards the central hole, with the second channel formed on an opposite side of the disc-shaped weight from the first channel;
wherein the first channel and the second channel are configured to receive therein a respective one of the protrusion or the another protrusion of the bottom support member.

12. The tool of any of claims 1-11, wherein the one or more weights comprises a plurality of weights stackable on the weight support pin, with each of the plurality of weights having an equal mass.

13. The tool of claim 12, wherein the downward force applied to the plunger rod is variable based on a number of the plurality of weights stacked on the weight support pin, with the downward force variable between 0.5 N and 5.0 N.

14. The tool of any of claims 1-13, wherein the lower tool portion comprises:
a main housing portion that defines a receptacle within which a portion of the syringe barrel is received;
an elastic gripper positioned within a portion of the receptacle adjacent an upper opening of the main housing portion, the elastic gripper configured to securely hold the syringe barrel, with an upper flange of the syringe barrel abutting a shoulder of the elastic gripper; and
a clamping nut positioned at an upper end of the main housing portion and over a portion of the elastic gripper, with the clamping nut secured to the main housing portion to retain the elastic gripper in place within the main housing portion, and with the shoulder extending up through an opening of the clamping nut.

15. The tool of any of claims 1-14, wherein the tool is configured to engage the plunger rod with the stopper via a twisting engagement of the plunger rod into the stopper.
